(19) Europäisches Patentamt
European Patent Office
Office européen des brevets

(11) Publication number: 0 030 734
B1

(12) **EUROPEAN PATENT SPECIFICATION**

(45) Date of publication of patent specification: 27.07.83

(21) Application number: 80107927.8

(22) Date of filing: 16.12.80

(51) Int. Cl.³: C 07 C 101/72, C 07 D 521/00, A 61 K 31/195 //C07C125/065, (C07D521/00, 213/51, 233/64, 417/12, 413/12, 409/12, 261/18, 285/08, 241/24, 403/12, 239/26, 307/54)

(54) **Tyrosine derivatives, a process for preparing the same and a pharmaceutical composition containing the same for treating hypertension.**

(30) Priority: 17.12.79 US 103975

(43) Date of publication of application: 24.06.81 Bulletin 81/25

(45) Publication of the grant of the patent: 27.07.83 Bulletin 83/30

(84) Designated Contracting States: AT BE CH DE FR GB IT LI LU NL SE

(56) References cited: US - A - 4 038 411

(73) Proprietor: MERCK & CO. INC.
126, East Lincoln Avenue P.O. Box 2000
Rahway New Jersey 07065 (US)

(72) Inventor: Baldwin, John J.
621 Gypsy Hill Circle
Gwynedd Valley Pennsylvania 19437 (US)
Inventor: Ponticello, Gerald S.
2045 Spring Valley Road
Lansdale Pennsylvania 19446 (US)
Inventor: Denny, George H.
125 Rosewood Drive
Lansdale Pennsylvania 19446 (US)
Inventor: Stone, Clement A.
450 Penllyn Pike
Blue Bell Pennsylvania 19422 (US)

(74) Representative: Abitz, Walter, Dr.-Ing. et al,
Abitz, Morf, Gritschneder P.O. Box 86 01 09
D-8000 München 86 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 030 734 B1

Tyrosine derivatives, a process for preparing the same and a pharmaceutical composition containing the same for treating hypertension

Background of the Invention:

The present invention concerns certain derivatives of tyrosine and their use as pharmaceuticals especially as antihypertensive agents.

Tyrosine is an $\alpha$-amino acid represented the formula

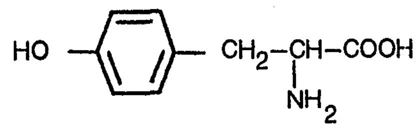

where —OH may be in the para position (p-tyrosine) meta position (m-tyrosine) or the ortho position (o-tyrosine). The L-isomer of p-tyrosine or its methyl ester when injected interperitoneally caused a reduction in blood pressure in test animals (rats) [Sved *et al.*, Proc. Natl. Acad. Sci. *76,* 3511—3514, July, (1979); Shalita *et al.*, Experientia *33,* 1430—1431 (1974)]. L-p-tyrosine, when fed orally to spontaneously hypertensive (SH) rats, was reported to cause a slight but non-continuing reduction in blood pressure [Osumi *et al.,* Japan J. Pharmacol. *24,* 715—720 (1974)]. m-tyrosine has antihypertensive activity in combination with a hydrazino decarboxylose inhibitor (U.S. 3,839,585). U.S. 4,038,411 teaches $\alpha$-methyl-3,4-dihydroxy-phenylalanine ester derivatives. No tyrosine esters are suggested.

Tyrosine derivatives have been discovered which are useful as hypertensive agents, particularly when orally administered.

Summary of the Invention

Tyrosine derivatives of the formula

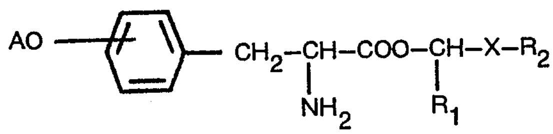

and use as pharmaceuticals.

Description of the Preferred Embodiments

An embodiment of the present invention is compounds of the formula

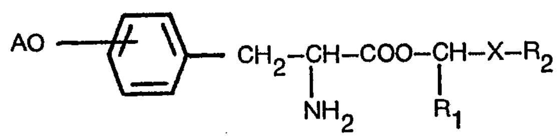

and pharmaceutically acceptable salts thereof wherein

A is H, $C_2$—$C_4$ alkanoyl, phen-$C_2$—$C_4$-alkanoyl, substituted phen-$C_2$—$C_4$-alkanoyl, benzoyl or substituted benzoyl,

$R_1$ is H, $C_1$—$C_4$-alkyl, phenyl, substituted phenyl, phen-$C_1$—$C_4$-alkyl, substituted phen-$C_1$—$C_4$-alkyl or a substituted or unsubstituted heteroaryl group having 5—6 ring members and 1—3 hetero atoms selected from O, N and S,

X is O or S, preferably O, and

$R_2$ is an acyl radical of the formula

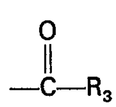

wherein

$R_3$ is $C_1$—$C_{17}$ alkyl, phenyl, substituted phenyl, phen-$C_1$—$C_4$-alkyl, substituted phen-$C_1$—$C_4$-alkyl, or heteroaryl having 5—6 ring members and 1—3 hetero atoms selected from O, S and N or said heteroaryl-$C_1$—$C_4$-alkyl, wherein all above substituents are halo, $C_{1-4}$ alkyl or $C_{1-4}$ alkoxy.

The pharmaceutically acceptable salts of the formula I compounds are the salt with appropriate and generally art recognized organic or inorganic acids. Suitable organic acids are the carboxylic acids exemplified by acetic acid, oxalic acid, pamoic acid, pivalic acid, fumaric acid, lauric acid, maleic acid, hexanoic acid, citric acid, and the like; and non-carboxylic acids such as isethionic acid, methanesulfonic acid and the like. The inorganic acids include the hydrohalides such as HCl, HBr and HI, phosphoric acid, sulfuric acid and the like.

The compound of formula I has a first chiral center at the —$NH_2$ bearing carbon atom and a second chiral center is possible at the —CH-position when the $R_1$ substituent is other than H. Thus, there are four possible stereoisomeric configurations of the formula I compound. The symbols used to designate the isomeric configurations are L and D, l and d, + and − and (R) and (S) or combinations thereof. Where no isomer designation is indicated for a formula or a named compound all the individual isomers, mixtures thereof and racemates are included. Where a designation is given e.g. (S), (R), L or D, it refers to the configuration at the first chiral center only.

The A substituent may be H, $C_2$—$C_4$-alkanoyl such as, acetyl or butanoyl, benzoyl, phen-$C_2$—$C_4$-alkanoyl such as

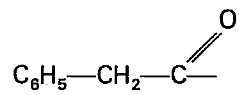

or

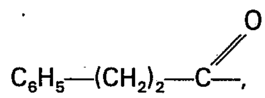,

substituted benzoyl or substituted phen-$C_2$—$C_4$-alkanoyl wherein the substituents are $C_1$—$C_4$ alkyl, $C_1$—$C_4$-alkoxy, or halo — the substituted phen-$C_2$—$C_4$-alkanoyl or benzoyl groups being illustrated by

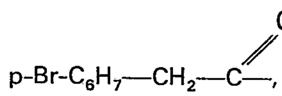,

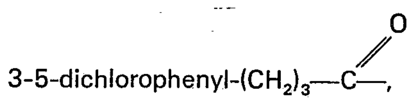,

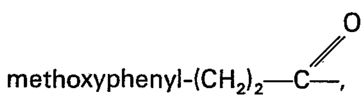,

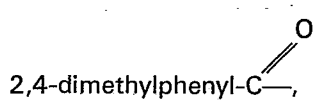,

and

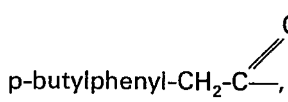,

the mono-substituted phenyl moiety being preferred. H is a more preferred definition of A.

The $R_1$ group includes H, $C_1$—$C_4$ alkyl such as methyl, isopropyl and butyl; phenyl; substituted phenyl wherein the substituents are halo, $C_1$—$C_4$ alkoxy or $C_1$—$C_4$ alkyl such as 2, 6 dibromophenyl, p-chlorophenyl, butoxyphenyl, 3,5-dimethylphenyl, isopropylphenyl, p-tolyl and p-chloro-o-tolyl, with the monosubstituted phenyl moiety being preferred; substituted phenyl moiety and phen-$C_1$—$C_4$-alkyl moieties are defined as above; and substituted or unsubstituted heteroaryl groups having 5—6 ring atoms containing 1—3 hetero atoms selected from O, S and N, said substituents being the same as those listed above for the phenyl moiety. Illustrative of the heteroaryl groups are pyridinyl, pyrazinyl, pyrimidinyl, pyridazinyl, 1,2,4-oxadiazolyl, 1,3,4-thiadiazolyl, oxazolyl, thiazolyl, Cl-pyridyl, isooxazolyl, isothiazolyl, imidiazolyl, triazolyl, thienyl, furyl and picolyl.

$R_2$ is an acyl radical

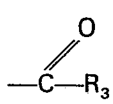

wherein

$R_3$ is as defined above. Illustrative of suitable $R_3$ groups is $CH_3$, hexyl, heptadecyl, l-methylpropyl, isopropyl, t-butyl and the like, phenyl, p-chlorophenyl, 2,4-dimethoxyphenyl, xylyl, isopropylphenyl and 2-bromo-4-ethoxyphenyl, imidazolyl, pyridyl, pyrazinyl, 1,3,4-thiadiazolyl, furyl, thienyl, oxazolyl, picolyl and thiazolyl.

Compounds of the formula

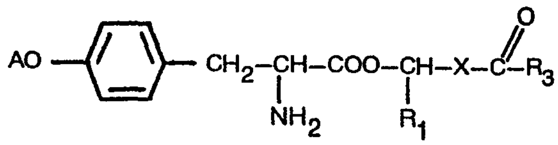

II

or

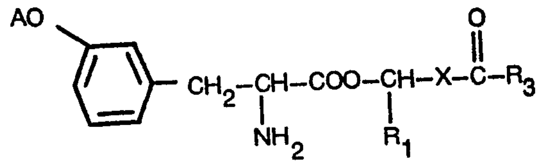

III

are preferred.

Another preferred group of compounds are those of Formula II or III when A is H, and especially where X is O.

A more preferred group of compounds has the formula

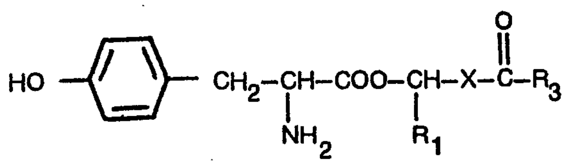

IV

wherein

$R_1$ is H or $C_1$—$C_3$ alkyl, phenyl and monosubstituted phenyl, and $R_3$ is $C_1$—$C_7$ alkyl, phenyl, monosubstituted phenyl, phen-$C_1$—$C_4$ alkyl and monosubstituted phen-$C_1$—$C_4$-alkyl, heteroaryl having 5—6 ring members selected from pyrazinyl, pyridyl, thienyl, furyl, pyridazinyl, thiazolyl, imidazolyl, 1,3,4-thiadiazolyl and picolyl, wherein the substituents are defined as above.

Particularly preferred compounds have the formula

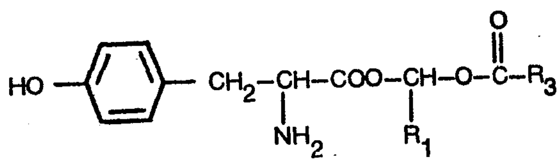

V

where $R_1$ is H or $CH_3$ and $R_3$ is $C_1$—$C_4$ alkyl, phenyl, monosubstituted phenyl, benzyl, monosubstituted benzyl, pyridyl, imidazolyl, picolyl or pyrazinyl, wherein the substituents are defined as above.

Especially preferred compounds have the formula

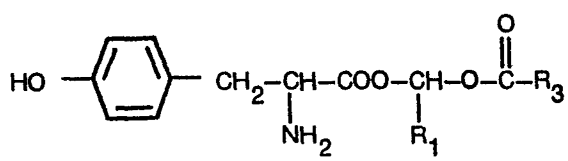

VI

wherein

$R_1$ is H or $CH_3$ and $R_3$ is $C_1$—$C_4$ alkyl. Compounds of the formula VI wherein $R_1$ is $CH_3$ are particularly preferred.

The compounds of formula I are useful for treating hypertension in human hypertensive patients when administered orally or parenterally. Oral administration is preferred. The daily dosage range may be varied as required. Useful daily dosage ranges are from 50 mg. to 3000 mg, preferably from 250 mg to 2000 mg. and more preferably from 250 mg. to 1500 mg.

For parenteral administration, appropriate dosage forms are exemplified by solutions, elixirs, dispersions and emulsions. For oral administration, suitable dosage forms are liquid e.g. solutions, emulsions, dispersions etc., as well as solid e.g. tablets, capsules and troches. The various dosage forms are prepared using conventional compounding procedures and ingredients, when necessary, such as diluents and carriers.

Compounds of the present invention may be prepared by any convenient process.

One such process is illustrated by the following set of equations:

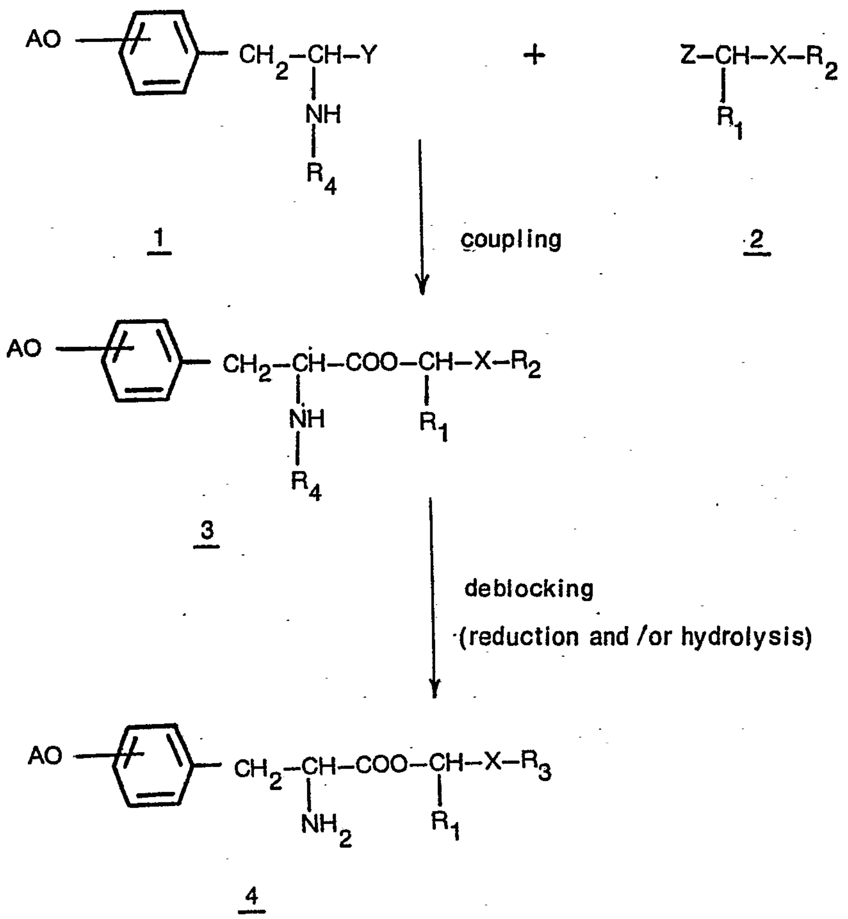


1 2 3 4

Compound *1* may have A as H or one of the other acyl groups defined above; X is O or S.

The $R_4$ group on the amino function is a blocking or protecting group and functions to protect the amino group from reacting in the reaction sequence. Any conventional N-blocking group may be used. Suitable groups are illustrated by carbobenzyloxy, p-methoxy-carbobenzyloxy, trifluroacetyl and t-butoxycarbonyl.

The group Y in intermediate *1* may be —COOH, or —COOM where M is an alkali metal.

Z in intermediate *2* is halogen e.g. Cl Br, especially Cl, or a substituted $—SO_3$-group, such as

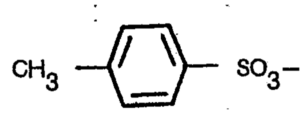

and nitrophenylsulfonyl.

The coupling reaction of *1* and *2* is carried out using conventional reaction conditions to obtain the intermediate *3*. Intermediate *3* is then appropriately "deblocked" to remove the $R_4$ protecting group to yield product *4*. The product *4* may be resolved into its isomers, if desired, by conventional means.

In a more preferred embodiment of the reaction sequence illustrated above, A is H and Y is halo, preferably Cl. Coupling (esterification) processes are illustrated in U.S. 3,983,138 and British Pat. 1,548,715, said teachings being incorporated herein by reference as necessary.

Intermediates of formula *2* where Z is halo may be prepared as illustrated by the following reaction equations

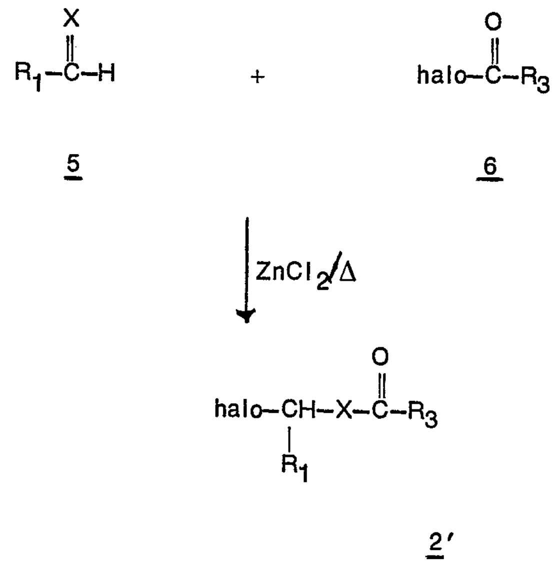


5 6 2′

This preparation involves reacting a suitable aldehyde (or thioaldehyde) *5* with a haloacyl compound *6* in fused $ZnCl_2$ under appropriate conditions.

The following Examples illustrate the preparation of representative compounds of the present invention.

## Example 1

A. 1-(2,2-Dimethyl-1-oxopropoxy)ethyl (S) α-[(phenylmethoxycarbonyl)amino]tyrosinate

To a solution of (S) α-[(phenylmethoxycarbonyl)amino]tyrosine (9.4 g, 0.03 mol) in dry DMF (50 ml) was added, under $N_2$, $Et_3N$ (4.2 ml., 0.03 mol.) followed by α-chloroethylpivalate. After stirring at 95°C for 6 hours, the reaction mixture was poured into $H_2O$ and the product extracted with EtOAc (3X). The organic layer was washed with saturated aq. $NaHCO_3$, saturated aq. NaCl, dried filtered and concentrated to dryness. The residue was chromatographed on silica gel and the product eluted with $CHCl_3$ to yield 6.4 g (59%) of 1-(2,2-dimethyl-1-oxopropoxy)-ethyl (S) α-[(phenylmethoxycarbonyl)-amino]tyrosinate.

B. 1-(2,2-Dimethyl-1-oxopropoxy)ethyl (S) tyrosinate hydrochloride hemihydrate

A suspension of 1-(2,2-dimethyl-1-oxopropoxy)ethyl (S) α-[(phenylmethoxycarbonyl)amino]-tyrosinate (6.4 g, 0.014 mol) in $CH_3OH$ (35 ml), EtOH (35 ml) and 8.85 *N* HCl EtOH (1.7 ml) was hydrogenated with 10% Pd/C catalyst (2 g) at an initial pressure of 3.2 bars for 6 hours. After removing catalyst by filtration, the solvents were removed under reduced pressure. The residue was covered with EtOAc—$Et_2O$ and stirred at room temperature to yield 3.4 g (67%) of 1-(2,2-dimethyl-1-oxopropoxy)ethyl (S) tyrosinate hydrochloride hemihydrate; m.p. 145—158°C.

## Example 2

1-(2,2-Dimethyl-1-oxopropoxy)methyl (S) tyrosinate hydrochloride

The preparation of 1-(2,2-dimethyl-1-oxopropoxy)methyl (S) tyrosinate hydrochloride was similar to the synthesis of 1-(2,2-dimethyl-1-oxopropoxy)ethyl (S) tyrosinate hydrochloride but using chloromethylpivalate in place of α-chloroethylpivalate; yield of 1-(2,2-dimethyl-1-oxopropoxy)methyl (S) tyrosinate hydrochloride was 76%, M.P. 135—140°C.

The next example illustrates an intermediate preparation.

## Example 3

Preparation of α-Chloroethylpivalate

Zinc chloride, 400 mg., was fused at 0.2—0.5 mm pressure and cooled to 25—30°C. under nitrogen. Pivaloyl chloride, 48 g. (0.40 mole) was added to the fused zinc chloride followed by acetaldehyde, 19.2 g. (0.44 mole). During addition of the acetaldehyde, which was done as rapidly as possible, the reaction mixture was stirred and cooled to prevent loss of acetaldehyde due to the exothermic nature of the reaction. After heating at reflux for 1 hour, distillation gave 36 g. (55%) of α-chloroethylpivalate, b.p. 32—34°C. at 5.42 mbars.

Other compounds, which may be prepared using the processes described herein or available in the art, are tabulated below.

TABLE 1

COMPOUNDS OF FORMULA

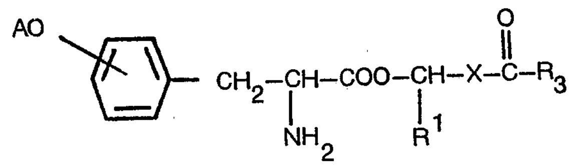

(1)

| Example | $R_1$ | X | $R_3$ | A |
|---|---|---|---|---|
| 4 | H | O | $CH_3-$ | H |
| 5 | $C_3H_7-$ | O | $C_{17}H_{35}-$ | H |
| 6 | $C_2H_5-$ | O | $C_7H_{15}-$ | H |
| 7 | $C_6H_5-$ | O | $-C(CH_3)_3$ | H |
| 8 | $Cl-C_6H_4-$ | O | $C_6H_5-$ | H |
| 9 | $CH_3-$ | O | $CH_3-$ | H |
| 10 | xyxlyl | O | $C_4H_9-O-C_6H_4-$ | H |
| 11 | $CH_3-O-C_6H_4-$ | O | $C_6H_5-CH_2-$ | $C_6H_5-CH_2-\overset{O}{\overset{\|}{C}}-$ |
| 12 | $C_2H_5-C_6H_4-(CH_2)_2-$ | O | $Cl-C_6H_4-CH_2-$ | $CH_3-\overset{O}{\overset{\|}{C}}-$ |
| 13 | 2-pyridyl | O | $CH_3O-C_6H_4(CH_2)_4-$ | $CH_3-(CH_2)_2-\overset{O}{\overset{\|}{C}}-$ |

TABLE 1 (Continued)

| Example | $R_1$ | X | $R_3$ | A |
|---|---|---|---|---|
| 14 | 2-imidazolyl | O | xylyl | $Cl-C_6H_4-\overset{O}{\overset{\|}{C}}-$ |
| 15 | 2-thiazolyl | O | imidazolyl$-CH_2-$ | 2,6-di$CH_3-C_6H_3-CH_2-\overset{O}{\overset{\|}{C}}-$ |
| 16 | 1,3,4-thiadiazolyl | O | 3-pyridyl | $C_6H_5-\overset{O}{\overset{\|}{C}}-$ |
| 17 | oxazolyl | O | picolyl | H |
| 18 | furyl | O | thienyl | H |
| 19 | thienyl | S | furyl | H |
| 20 | picolyl | S | thiazolyl | H |
| 21 | $CH_3-$ | S | isooxazolyl | H |
| 22 | H | S | 1,3,5 thiadiazolyl | H |
| 23 | $CH_3-$ | S | pyrazinyl | $CH_3-CH_2-\overset{O}{\overset{\|}{C}}-$ |
| 24 | pyrazinyl | S | pyridazinyl | tolyl$-(CH_2)_3-\overset{O}{\overset{\|}{C}}-$ |
| 25 | tolyl | S | $C_2H_5-$ | $CH_3O-C_6H_4-CH_2-\overset{O}{\overset{\|}{C}}-$ |
| 26 | pyrimidinyl | S | $C_4H_9-$ | H |

TABLE 1 (Continued)

| Example | $R_1$ | X | $R_3$ | A |
|---|---|---|---|---|
| 27 | $CH_3$ | S | furyl–$(CH_2)_4$– | $C_6H_5-\overset{O}{\overset{\|}{C}}-$ |
| 28 | isothiazolyl | O | triazolyl | 3,5–diBr–$C_6H_3-CH_2-\overset{O}{\overset{\|}{C}}-$ |

AO– is o, m or p; preferably m- or p- and most preferably p-.

**Claims for the contracting states: BE CH DE FR GB IT LI LU NL SE**

1. Compounds having the formula:

a)

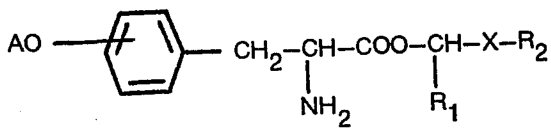

(I)

wherein

A is H, $C_2$—$C_4$-alkanoyl, benzoyl, substituted benzoyl, phen-$C_2$—$C_4$ alkanoyl or substituted phen-$C_2$—$C_4$-alkanoyl,

$R_1$ is H, $C_1$—$C_4$ alkyl, phenyl, substituted phenyl, phen-$C_1$—$C_4$-alkyl, substituted phen-$C_1$—$C_4$-alkyl and substituted or unsubstituted heteroaryl group having 5—6 ring members and 1—3 hetero atoms selected from O, N and S,

X is O or S and

$R_2$ is an acyl radical of the formula

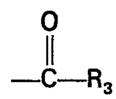

wherein

$R_3$ is $C_1$—$C_{17}$ alkyl, phenyl, substituted phenyl, phen-$C_1$—$C_4$-alkyl, substituted phen-$C_1$—$C_4$-alkyl, heteroaryl having 5—6 ring members and 1—3 hetero atoms selected from O, S and N or said heteroaryl-$C_1$—$C_4$-alkyl, wherein all substituents are halo, $C_{1-4}$ alkyl or $C_{1-4}$ alkoxy,

b) the compound of Formula I wherein A is H,

c) the compound of Formula I wherein A is H and having the D-isomer configuration, or

d) the compound of Formula I wherein A is H and having the L-isomer configuration.

2. The compounds of Claim 1, general Formula I

i) having the specific formula:

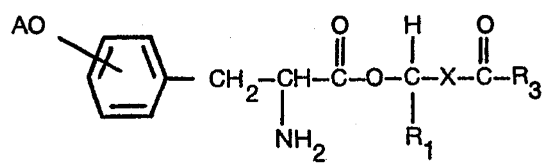

(II)

or

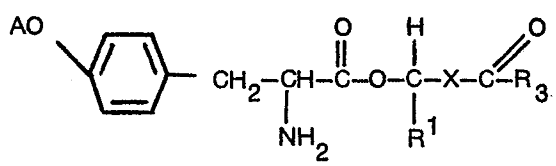

(III)

ii) the compounds of Formula III wherein X is S,

iii) the compounds of Formula III wherein X is O,

iv) the compounds of Formula III wherein A is H and X is O

(v) the compounds of Formula III wherein $R_3$ is alkyl, A is H, and X is O or

vi) the compounds of Formula III wherein $R_3$ is $C(CH_3)_3$, A is H, and X is O.

3. The compounds of Claim 1, Formula I having the specific formula:

A)

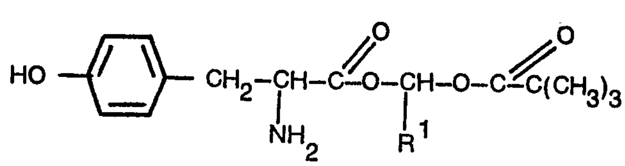

(IV)

wherein

$R_1$ is H or $CH_3$,

B) the compounds of Formula IV wherein $R^1$ is $CH_3$,

C) the compounds of Formula IV having the D-isomer configuration,

D) the compounds of Formula IV having the L-isomer configuration,

E) the compound of Formula IV wherein $R^1$ is $CH_3$ and having the D-isomer configuration or

F) the compound of Formula IV wherein $R^1$ is $CH_3$ and having the L-isomer configuration.

4. A pharmaceutical composition for treating hypertension in human beings containing an effective amount of a compound of Claim 1a).

5. A process for preparing compounds of the formula I according to Claim 1 which comprises

a) coupling of a compound of the formula

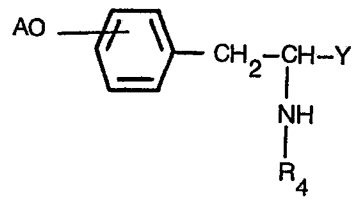

with a compound of the formula

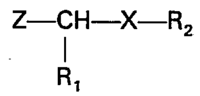

wherein

Y is COOH or COOM where

M is alkali metal,

Z is a halogen or a substituted $—SO_3—$ group and

$R_4$ is an amino protecting group, to obtain a compound of the formula:

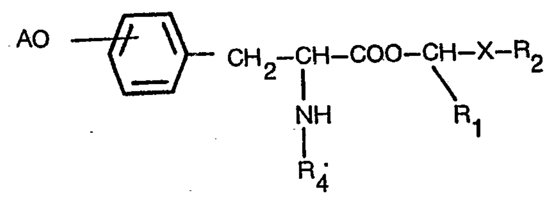

(V)

b) removing the $R_4$ protecting group from the Formula V compound to obtain the formula I compound.

**Claim for the contracting state: AT**

A process for preparing compounds of the formula:

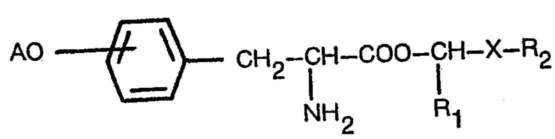

(I)

wherein

A is H, $C_2$—$C_4$-alkanoyl, benzoyl, substituted benzoyl, phen-$C_2$—$C_4$ alkanoyl or substituted phen-$C_2$—$C_4$-alkanoyl,

$R_1$ is H, $C_1$—$C_4$ alkyl, phenyl, substituted phenyl, phen-$C_1$—$C_4$-alkyl, substituted phen-$C_1$—$C_4$-alkyl and substituted or unsubstituted heteroaryl group having 5—6 ring members and 1—3 hetero atoms selected from O, N and S,

X is O or S and

$R_2$ is an acyl radical of the formula

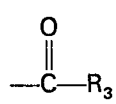

wherein

$R_3$ is $C_1$—$C_{17}$ alkyl, phenyl, substituted phenyl, phen-$C_1$—$C_4$-alkyl, substituted phen-$C_1$—$C_4$-alkyl, heteroaryl having 5—6 ring members and 1—3 hetero atoms selected from O, S and N or said heteroaryl-$C_1$—$C_4$-alkyl, wherein all substituents are halo, $C_{1-4}$ alkyl or $C_{1-4}$ alkoxy, which comprises

a) coupling of a compound of the formula

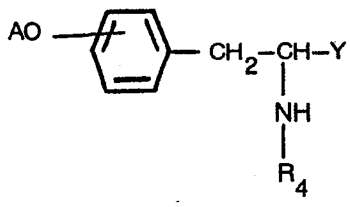

with a compound of the formula

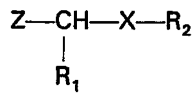

wherein

Y is COOH or COOM where

M is alkali metal,

Z is a halogen or a substituted $—SO_3—$ group and

$R_4$ is an amino protecting group, to obtain a compound of the formula:

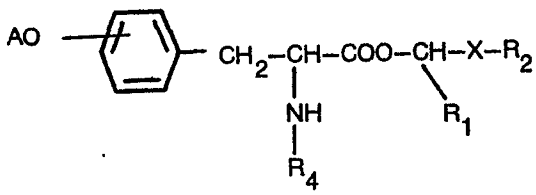

(V)

b) removing the $R_4$ protecting group from the Formula V compound to obtain the formula I compound.

**Revendications pour les etats contractants: BE CH DE FR GB IT LI LU NL SE**

1. Composés de formule:

a)

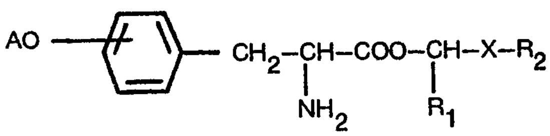

I

où

A représente H, un alcanoyle en $C_2$ à $C_4$, un phén-alcanoyle en $C_2$ à $C_4$, un phén-alcanoyle en $C_2$ à $C_4$ substitué, un benzoyle ou un benzoyle substitué,

$R_1$ représente H, un alcoyle en $C_1$ à $C_4$, un phényle, un phényle substitué, un phén-alcoyle en $C_1$ à $C_4$, un phén-alcoyle en $C_1$ à $C_4$ substitué et un groupe hétéro-aryle substitué ou non substitué ayant 5 à 6 chaînons dans son noyau et de 1 à 3 hétéro-atomes choisis entre O, N et S,

X représente O ou S, et

$R_2$ est un radical acyle de formule

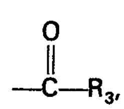

où $R_3$ est un alcoyle en $C_1$ à $C_{17}$, un phényle, un phényle substitué, un phén-alcoyle en $C_1$ à $C_4$, un phén-alcoyle en $C_1$ à $C_4$ substitué, un hétéro-aryle ayant 5 à 6 chaînons dans son noyau et de 1 à 3 hétéro-atomes choisis entre O, S et N ou ledit hétéro-aryl-alcoyle en $C_1$ à $C_4$, où tous les substituants ci-dessus sont des halogènes, des alcoyle en $C_1$ à $C_4$ ou des alcoxy en $C_1$ à $C_4$,

b) le composé de formule I où A est H,

c) le composé de formule I où A est H et qui a la configuration isomérique D, ou

d) le composé de formule I où A est H et qui a la configuration isomérique L.

2. Composés selon la revendication 1, de formule générale I

i) répondant à la formule spécifique:

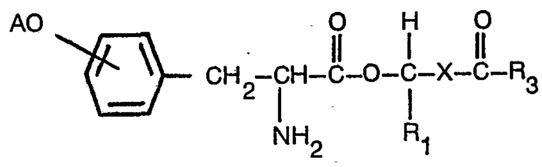

II

ou

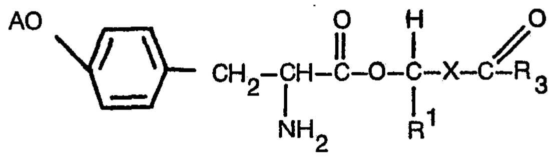

III

ii) les composés de formule III où X est S,
iii) les composés de formule III où X est O,
iv) les composés de formule III où A est H et X est O,
v) les composés de formule III où $R_3$ est un alcoyle, A est H, et X est O ou
vi) les composés de formule III où $R_3$ est $C(CH_3)_3$, A est H et X est O.

3. Composés selon la revendication 1 de la formule I ayant la formule spécifique:

A)

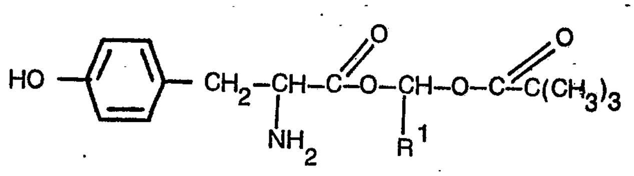

IV

où $R_1$ est H ou $CH_3$,

B) les composés de formule IV où $R_1$ est $CH_3$,
C) les composés de formule IV ayant la configuration isomérique D,
D) les composés de formule IV ayant la configuration isomérique L,
E) le composé de formule IV où $R_1$ est $CH_3$ et qui a la configuration isomérique D ou
F) le composé de formule IV où $R_1$ est $CH_3$ et qui a la configuration isomérique L.

4. Composition pharmaceutique pour traiter l'hypertension chez l'homme, contenant une quantité efficace d'un composé selon la revendication 1a).

5. Procédé de préparation de composés de formule I selon la revendication 1 dans lequel

a) on couple un composé de formule:

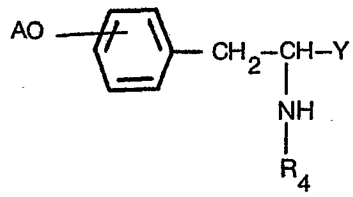

avec un composé de formule:

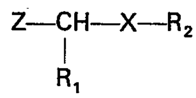

où

Y est COOH ou COOM où
M est un métal alcalin,
Z est un halogène ou un groupe $—SO_3$ substitué et
$R_4$ est un groupe protecteur d'amino pour obtenir un composé de formule:

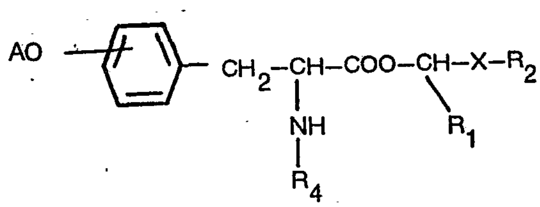

V

b) on enlève le groupe protecteur $R_4$ du composé de formule V pour obtenir le composé de formule I.

**Revendication pour l'etat contractant: AT**

Procédé de préparation de composés de formule:

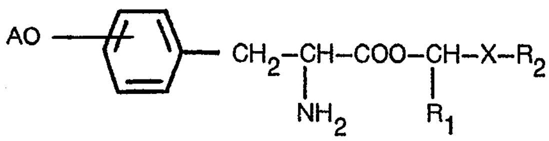

I

où

A représente H, un alcanoyle en $C_2$ à $C_4$, un phén-alcanoyle en $C_2$ à $C_4$, un phén-alcanoyle en $C_2$ à $C_4$ substitué, un benzoyle ou un benzoyle substitué,

$R_1$ représente H, un alcoyle en $C_1$ à $C_4$, un phényle, un phényle substitué, un phén-alcoyle en $C_1$ à $C_4$, un phén-alcoyle en $C_1$ à $C_4$ substitué et un groupe hétéro-aryle substitué ou non substitué ayant 5 à 6 chaînons dans son noyau et de 1 à 3 hétéro-atomes choisis entre O, N et S,

X représente O ou S, et

$R_2$ est un radical acyle de formule

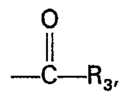

où $R_3$ est un alcoyle en $C_1$ à $C_{17}$, un phényle, un phényle substitué, un phén-alcoyle en $C_1$ à $C_4$, un phén-alcoyle en $C_1$ à $C_4$ substitué, un hétéro-aryle ayant 5 à 6 chaînons dans son noyau et de 1 à 3 hétéro-atomes choisis entre O, S et N ou ledit hétéro-aryle-alcoyle en $C_1$ à $C_4$, où tous les substituants ci-dessus sont des halogènes, des alcoyles en $C_1$ à $C_4$ ou des alcoxy en $C_1$ à $C_4$ dans lequel:

a) on couple un composé de formule:

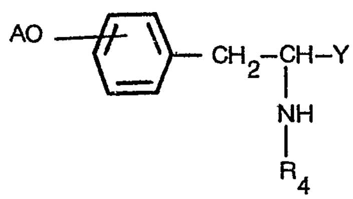

avec un composé de formule:

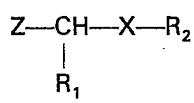

où

Y est COOH ou COOM où

M est un métal alcalin,

Z est un halogène ou un groupe $—SO_3$ substitué et

$R_4$ est un groupe protecteur d'amino, pour obtenir un composé de formule:

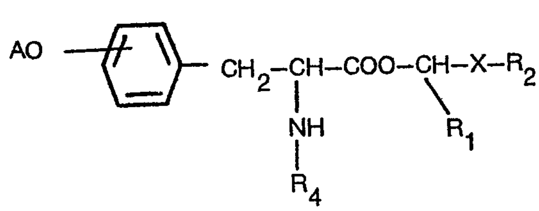

V

b) on retire le groupe protecteur $R_4$ du composé de formule V pour obtenir le composé de formule I.

**Patentansprüche für die Vertragsstaaten: BE CH DE FR GB IT LI LU NL SE**

1. Verbindungen der Formel
a)

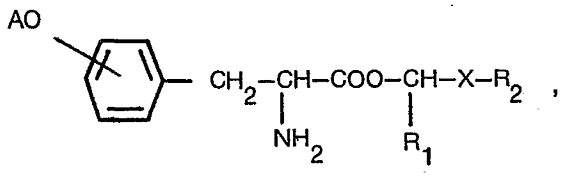

(I)

worin
A H, $C_2$—$C_4$-Alkanoyl, Benzoyl, substituiertes Benzoyl, Phen-$C_2$—$C_4$-alkanoyl oder substituiertes Phen-$C_2$—$C_4$-alkanoyl ist,
$R_1$ H, $C_1$—$C_4$-Alkyl, Phenyl, substituiertes Phenyl, Phen-$C_1$—$C_4$-alkyl, substituiertes Phen-$C_1$—$C_4$-alkyl und eine substituierte oder unsubstituierte Heteroarylgruppe mit 5—6 Ringgliedern und 1—3 aus O, N und S ausgewählten Heteroatomen ist,
X O oder S ist und
$R_2$ ein Acylrest der Formel

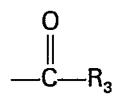

ist, worin $R_3$ $C_1$—$C_{17}$-Alkyl, Phenyl, substituiertes Phenyl, Phen-$C_1$—$C_4$-alkyl, substituiertes Phen-$C_1$—$C_4$-alkyl, Heteroaryl mit 5—6 Ringgliedern und 1—3 aus O, S und N ausgewählten Heteroatomen oder das genannte Heteroaryl-$C_1$—$C_4$-alkyl ist, worin alle Substituenten Halogen, $C_{1-4}$-Alkyl oder $C_{1-4}$-Alkoxy sind;
b) die Verbindung der Formel I, worin A H ist;
c) die Verbindung der Formel I, worin A H ist und die die D-Isomerkonfiguration hat, oder
d) die Verbindung der Formel I, worin A H ist und die die L-Isomerkonfiguration hat.

2. Die Verbindungen nach Anspruch 1, allgemeine Formel I,
i) welche die spezielle Formel:

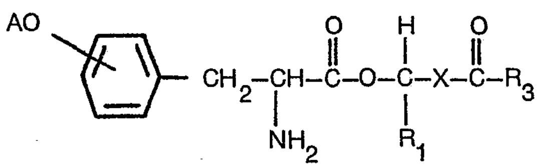

(II)

oder

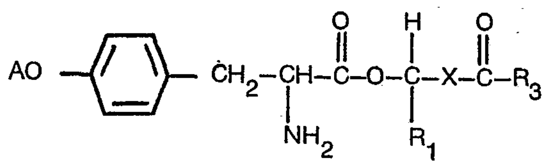

(III)

haben,
ii) die Verbindungen der Formel III, worin X S ist,
iii) die Verbindungen der Formel III, worin X O ist,
iv) die Verbindungen der Formel III, worin A H ist und X O ist,
v) die Verbindungen der Formel III, worin $R_3$ Alkyl ist, A H ist und X O ist oder
vi) die Verbindungen der Formel III, worin $R_3$ $C(CH_3)_3$ ist, A H ist und X O ist.

3. Die Verbindungen nach Anspruch 1, Formel I, welche die spezielle Formel:
A)

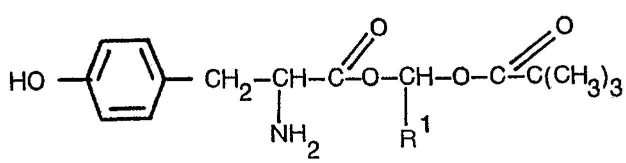

(IV)

haben, worin $R_1$ H oder $CH_3$ ist,
B) die Verbindungen der Formel IV, worin $R_1$ $CH_3$ ist,
C) die Verbindungen der Formel IV, welche die D-Isomerkonfiguration haben,
D) die Verbindungen der Formel IV, welche die L-Isomerkonfiguration haben,
E) die Verbindung der Formel IV, worin $R_1$ $CH_3$ ist und die die D-Isomerkonfiguration hat oder

F) die Verbindung der Formel IV, worin $R_1$ $CH_3$ ist und die die L-Konfiguration hat.

4. Eine pharmazeutische Zusammensetzung zur Behandlung von Hochdruck beim Menschen, enthaltend eine wirksame Menge einer Verbindung nach Anspruch 1a).

5. Ein Verfahren zur Herstellung von Verbindungen der Formel I nach Anspruch 1, umfassend

a) das Kuppeln einer Verbindung der Formel

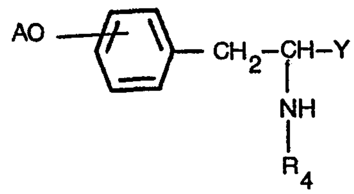

mit einer Verbindung der Formel

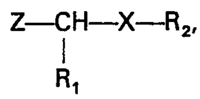

worin

Y COOH oder COOM ist, wobei

M ein Alkalimetall ist,

Z ein Halogen oder eine substituierte $—SO_3$-Gruppe ist und

$R_4$ eine Aminoschutzgruppe ist, um eine Verbindung der Formel:

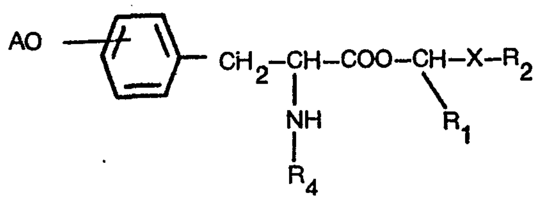

(V)

zu erhalten,

b) das Entfernen der $R_4$-Schutzgruppe aus der Verbindung der Formel V, um die Verbindung der Formel I zu erhalten.

**Patentanspruch für den Vertragsstaat: AT**

Ein Verfahren zur Herstellung von Verbindungen der Formel:

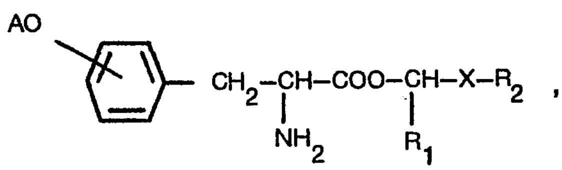

(I)

worin

A H, $C_2$—$C_4$-Alkanoyl, Benzoyl, substituiertes Benzoyl, Phen-$C_2$—$C_4$-alkanoyl oder substituiertes Phen-$C_2$—$C_4$-alkanoyl ist,

$R_1$ H, $C_1$—$C_4$-Alkyl, Phenyl, substituiertes Phenyl, Phen-$C_1$—$C_4$-alkyl, substituiertes Phen-$C_1$—$C_4$-alkyl und eine substituierte oder unsubstituierte Heteroarylgruppe mit 5—6 Ringgliedern und 1—3 aus O, N und S ausgewählten Heteroatomen ist,

X O oder S ist und

$R_2$ ein Acylrest der Formel

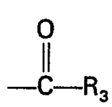

ist, worin $R_3$ $C_1$—$C_{17}$-Alkyl, Phenyl, substituiertes Phenyl, Phen-$C_1$—$C_4$-alkyl, substituiertes Phen-$C_1$—$C_4$-alkyl, Heteroaryl mit 5—6 Ringgliedern und 1—3 aus O, S und N ausgewählten Heteroatomen oder das erwähnte Heteroaryl-$C_1$—$C_4$-alkyl ist, worin alle Substituenten Halogen, $C_{1-4}$-Alkyl oder $C_{1-4}$-Alkoxy sind, umfassend

a) das Kuppeln einer Verbindung der Formel

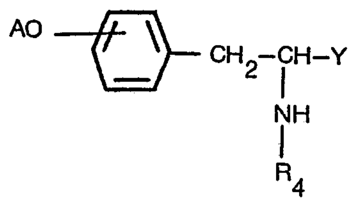

mit einer Verbindung der Formel

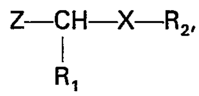

worin

Y COOH oder COOM ist, wobei

M Alkalimetall ist,

Z ein Halogen oder eine substituierte $—SO_3$-Gruppe ist und

$R_4$ eine Aminoschutzgruppe ist, um eine Verbindung der Formel:

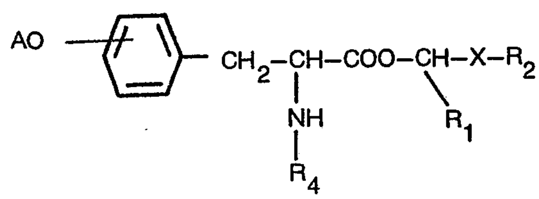

(V)

zu erhalten,

b) das Entfernen der $R_4$-Schutzgruppe aus der Verbindung der Formel V, um die Verbindung der Formel I zu erhalten.